Europäisches Patentamt

European Patent Office

Office européen des brevets

(19)

(11) Publication number: **0 335 627**
A2

(12)

# EUROPEAN PATENT APPLICATION

(21) Application number: 89302990.0

(22) Date of filing: 28.03.89

(51) Int. Cl.⁴: **A 61 K 37/02**
// (A61K37/02,31:445)

(30) Priority: 29.03.88 GB 8807505

(43) Date of publication of application:
04.10.89 Bulletin 89/40

(84) Designated Contracting States:
AT BE CH DE FR GB IT LI NL SE

(71) Applicant: GLAXO GROUP LIMITED
Clarges House 6-12 Clarges Street
London W1Y 8DH (GB)

(72) Inventor: McAllister, Anthony
4 Redwoods, Bengeo
Hertford, Herts SG14 3ET (GB)

(74) Representative: Holmes, Michael John et al
Frank B. Dehn & Co. Imperial House 15-19 Kingsway
London WC2B 6UZ (GB)

(54) Use of a thromboxane antagonist in cyclosporin A-induced nephrotoxicity.

(57) The use of the thromboxane antagonists [1R-[1α(Z),2β,3β,5α]]-(+)-7-[5[[(1,1'-biphenyl)-4-yl]-methoxy]-3-hydroxy-2-(1-piperidinyl) cyclopentyl]-4-heptenoic acid in the treatment of cyclosporin A induced nephrotoxicity.

EP 0 335 627 A2

**Description**

## USE OF A THROMBOXANE ANTAGONIST IN CYCLOSPORIN A-INDUCED NEPHROTOXICITY

The present invention relates to a combination of cyclosporin A with [1R-[1α(Z),2β,3β,5α]]-(+)-7-[5-[[(1,1'-biphenyl)-4-yl]methoxy]-3-hydroxy-2(1-piperidinyl)cyclopentyl]-4-heptenoic acid (hereinafter referred to as Compound A) or a physiologically acceptable salt, solvate or cyclodextrin complex thereof, to pharmaceutical formulations containing them, and to their use in human or veterinary medicine.

Cyclosporin A, a metabolite of the fungi Cylindrocarpon lucidum Booth and Trichoderma polysporum, is a cyclic polypeptide composed of eleven amino acids. The compound is a promising immunosuppressive agent and is now widely used for prolonging the function of various transplanted organs. In addition to its use in graft recipients, cyclosporin A may also be of use in the management of a wide range of autoimmune diseases, including psoriasis, polymyositis, multiple sclerosis, ureitus posterior, primary biliary cirrhosis, systemic lupus erythematosis, rheumatoid arthritis, endocrine ophthalmopathy and early insulin dependent diabetes. The compound also has interesting anti-schistosome and anti-malarial activities.

Despite its great promise as an immunosuppressive, the clinical use of cyclosporin A is somewhat limited both by its association with infection and also because of hepatic and renal toxicities. Bennett W. M., et. al., Ann. Int. Med., 99, 851-854 (1983) have pointed out the substantial nephrotoxic potential accompanying cyclosporin A therapy in patients receiving kidney, heart, bone marrow and liver transplants. Indeed, some nephrotoxicity is reported to occur in almost 80% of renal transplant patients using cyclosporin A. Frequent side effects reported for cyclosporin A treatment in various autoimmune diseases include nephrotoxicity, hypertension, hyperkalemia, hyperuricemia, hepatoxicity, anaemia, hypertrichosis, gingival hyperplasia, gastrointestinal intolerance and paresthesia.

Our UK Patent Specification 2097397 describes inter alia Compound A which is a potent thromboxane receptor blocker. We have stated therein that the compounds of the invention, including Compound A, inhibit thromboxane A₂ and endoperoxide mediated aggregration of blood platelets and contraction of vascular smooth muscle and are thus of particular interest as anti-thrombotic agents.

We have now found that Compound A or a physiologically acceptable salt, solvate or cyclodextrin complex thereof is of value in reducing side effects associated with cyclosporin A administration. Compound A or a physiologically acceptable salt, solvate or cyclodextrin complex thereof and cyclosporin A in combination therapy is therefore of interest for use in human and veterinary medicine, more particularly for use in the management of graft rejection and autoimmune diseases. Compound A or a physiologically acceptable salt, solvate or cyclodextrin complex thereof is of particular benefit in reducing the nephrotoxicity caused by administration of cyclosporin A.

Thus, according to one aspect of the invention, we provide a method for reducing side effects (e.g. the nephrotoxicity) caused by cyclosporin A comprising administering to the patient an amount of Compound A or a physiologically acceptable salt, solvate or cyclodextrin complex thereof sufficient to reduce side effects (e.g. the nephrotoxicity) caused by said cyclosporin A.

It will be appreciated that cyclosporin A and Compound A or a physiologically acceptable salt, solvate or cyclodextrin complex thereof may be administered to the patient either in combination or separately. If the two compounds are administered separately Compound A or a physiologically acceptable salt, solvate or cyclodextrin complex thereof will preferably be administered first, and the administration should be managed such that Compound A or a physiologically acceptable salt, solvate or cyclodextrin complex thereof will have an effect in the human or animal body in reducing side effects (e.g. the nephrotoxicity) caused by cyclosporin A

In another aspect of the invention, therefore, we provide cyclosporin A and Compound A or a physiologically acceptable salt, solvate or cyclodextrin complex thereof in the presence of each other in the patient for use in human or veterinary medicine, more particularly for use in the management of graft rejection and autoimmune diseases, especially in the management of graft rejection and the treatment of psoriasis and early insulin dependent diabetes.

In a further aspect of the invention we provide a combination of cyclosporin A and Compound A or a physiologically acceptable salt, solvate or cyclodextrin complex thereof.

In a yet further aspect of the invention we provide a combination of cyclosporin A and Compound A or a physiologically acceptable salt, solvate or cyclodextrin complex thereof for use in human or veterinary medicine, more particularly for use in the management of graft rejection and autoimmune diseases, especially in the management of graft rejection and the treatment of psoriasis and early insulin dependent diabetes.

In another aspect of the invention we provide the use of cyclosporin A and Compound A or a physiologically acceptable salt, solvate or cyclodextrin complex thereof in the preparation of a medicament for the management of graft rejection and autoimmune diseases, especially in the management of graft rejection and the treatment of psoriasis and early insulin dependent diabetes, in the human or animal body.

In using cyclosporin A and compound A or a physiologically acceptable salt, solvate or cyclodextrin complex thereof accord to the present invention, it is preferable to employ them in the form of formulations which may be in separate formulations or in a single combined formulation. However, in the latter formulation both active ingredients must of course be stable and mutually compatable in the particular formulaiton employed.

The present invention therefore also provides a pharmaceutical composition which comprises cyclosporin A and Compound A or a physiologically acceptable salt, solvate or cyclodextrin complex thereof together, where desirable, with one or more pharmaceutical carriers or excipients.

In another aspect of the invention we provide a pharmaceutical composition comprising cyclosporin A and Compound A or a physiologically acceptable salt, solvate or cyclodextrin complex thereof together, where desirable, with one or more pharmaceutical carriers or excipient for use in human or veterinary medicine, more particularly for use in the management of graft rejection and autoimmune diseases, especially in the management of graft rejection and the treatment of psoriasis and early insulin dependent diabetes.

The single combined composition may be prepared by admixture of the active ingredients together, where desirable, with one or more pharmaceutical carriers or excipients. Thus, in another aspect of the invention we provide a process for the preparation of a pharmaceutical composition which comprises mixing cyclosporin A and Compound A or a physiologically acceptable salt, solvate or cyclodextrin complex thereof together, where desirable, with one or more pharmaceutical carriers or excipients.

When cyclosporin A and Compound A or a physiologically acceptable salt, solvate or cyclodextrin complex thereof are to be used in combination, the combination may be administered by any suitable method known in the medicinal arts. Suitable modes of administration include, but are not limited to, oral parenteral (e.g. intramuscular, subcutaneous, intraperitoneal or intravenous) administration.

Formulations containing cyclosporin A and Compound A or a physiologically acceptable salt, solvate or cyclodextrin complex thereof in a single combined composition may be prepared in a similar manner to the preparation of formulations suitable for cyclosporin A or Compound A or a physiologically acceptable salt, solvate or cyclodextrin complex thereof per se. Suitable preparations include, but are not limited to, suspensions, solutions, emulsions and tablets. Thus, for example, parenteral formulations may may be presented in the form of suspensions, solutions or emulsions in oil or aqueous vehicles which may optionally contain formulatory agents such as suspending, stabilising and/or dispersing agents. Alternatively, the active ingredients may be in powder form for reconstitution before use with a suitable vehicle. Formulations for injections may be presented in unit dosage form in ampoules, or in a multidose container with an added preservative, or in a suitable container for infusion.

As stated hereinbefore, cyclosporin A and Compound A or a physiologically acceptable salt, solvate or cyclodextrin complex thereof may be administered as two separate compositions. Cyclosporin A will generally be in a form suitable for parenteral or oral administration. Similarly, Compound A or a physiologically acceptable salt, solvate or cyclodextrin complex thereof may be used in various pharmaceutical formulations for oral or parenteral administration.

It may be convenient to present cyclosporin A and Compound A or a physiologically acceptable salt, solvate or cyclodextrin complex thereof as a two container pack, one container containing cyclosporin A and the other containing Compound A or a physiologically acceptable salt, solvate or cyclodextrin complex thereof. The compounds may then be admixed immediately before administration or, if desired, may be administered separately either simultaneously or sequentially. If the compounds are administered separately Compound A or a physiologically acceptable salt, solvate or cyclodextrin complex thereof will preferably be administered first.

The invention also provides cyclosporin A and Compound A or a physiologically acceptable salt, solvate or cyclodextrin complex thereof and compositions containing them, in association with instructions for their use together in human or veterinary medicine, more particularly for their use together in the management of graft rejection and autoimmune diseases, especially in the management of graft rejection and the treatment of psoriasis and early insulin dependent diabetes.

As mentioned hereinbefore, cyclosporin A is commercially available, and pharmaceutical formulations containing cyclosporin A are known in the medicinal arts.

Suitable salts of Compound A include acid addition salts derived from inorganic and organic acids such as hydrochlorides, hyrobromides, sulphates, phosphates, maleates, tartrates, citrates, benzoates, 2-chlorobenzoates, p-toluenesulphonates, methanesulphonates salicylates, fumarates, lactates, hydroxynaphthalenecarboxylates (e.g. 1-hydroxy- or 3-hydroxy-2-naphthalenecarboxylates) or furoates, or salts with suitable bases such as alkali metal (e.g. sodium and potassium), alkaline earth metal (e.g. calcium or magnesium), ammonium and substituted ammonium (e.g. dimethylammonium, triethylammonium, 2-hydroxyethyl dimethylammonium, piperazine, N,N-dimethylpiperazine, piperidine, ethylenediamine and choline) salts. A preferred salt of Compound A is the hydrochloride salt. Compound A and physiologically acceptable salts and solvates thereof and pharmaceutical formulations containing them are described in GB-B-2097397 and GB-B-2127406.

When Compound A is used according to the present invention in the form of a cyclodextrin complex, the complex conveniently contains a molar ratio of Compound A with cyclodextrin within the range 1:1 to 1:3. The cyclodextrin within the complex amy be any of $\alpha$-, $\beta$- or $\gamma$-cyclodextrin or a mixture of any of two or three of them, although preferably the complex contains $\beta$-cyclodextrin. A particularly preferred cyclodextrin complex of Compound A is the $\beta$-cyclodextrin complex in which the molar ratio of Compound A with $\beta$-cyclodextrin is about 1:1.

Cyclodextrin complexes of Compound A and pharmaceutical formulations containing them are described in our co-pending UK Patent Applications

Nos. 3729823 and 8804422. The cyclodextrin complexes may be prepared by dissolving Compound A or the hydrochloride salt thereof in water or an organic solvent which is miscible with water (e.g. an alcohol such as methanol) and adding to the solution a solution of $\alpha$-, $\beta$- or $\gamma$-cyclodextrin (or a hydrate thereof) or a mixture of two or three of them together in water and/or an organic solvent which is miscible with water. The reaction may take place at any temperature in the range from 0° to 80°C. However, the mixture is preferably kept at room temperature and the desired complex obtained by concentrating the mixture under reduced pressure or by allowing the mixture to cool. The mixing ratio of organic solvent with water may be suitably varied according to the solubilities of the starting materials and products. Preferably 1 to 4 moles of cyclodextrin are used for each mole of Compound A or its hydrochloride salt.

Pharmaceutical formulations containing a cyclodextrin complex of Compound A may be prepared in conventional manner by mixing the complex with one or more pharmaceutical carriers or excipients, for example, according to the general methods described in GB-B-2097397 and GB-B-2127406.

When Compound A or a physiologically acceptable salt, solvate or cyclodextrin complex is to be administered as a liquid formulation for, in particular, parenteral (e.g. intravenous) use, the composition may be prepared according to the general methods described in GB-B-2097397 and GB-B-2127406. Alternatively, the liquid compositions may be prepared by mixing Compound A or the hydrochloride salt thereof with $\alpha$-, $\beta$- or $\gamma$-cyclodextrin (or a hydrate thereof) or a mixture of two or three of them together, where desirable, with one or more pharmaceutical carriers of excipients in a suitable medium such as water according to conventional methods. The molar ratio of Compound A or its hydrochloride salt with cyclodextrin in the liquid composition is conveniently within the range 1:1 to 1:4, and preferably the liquid composition is an aqueous solution which contains the hydrochloride salt of Compound A and $\beta$-cyclodextrin (or a hydrate thereof) in a molar ratio of about 1:1.4.

The precise dose of cyclosporin A and Compound A or a physiologically acceptable salt, solvate or cyclodextrin complex thereof to be administered and the length of the course of treatment will, of course, depend on a number of factors including, for example, the age and weight of the patient, the specific conditions requiring treatment and its severity and the route of administration. An effective dose, however, in the case of Compound A or a physiologically acceptable salt, solvate or cyclodextrin complex thereof is likely to be in the range from 0.05 to 5 mg/kg body weight of patient per day, preferably in the range from 0.05 to 1 mg/kg per day. Dosages and dosage rates in the case of cyclosporin A will generally correspond with dosages and dosage rates one would use if administering cyclosporin A alone to treat the human or animal patient. Effective dose regimes for cyclosporin A administration are now well-established. However, a typical therapeutic amount of cyclosporin A given orally or intramuscularly usually ranges from 5 to 25mg/kg body weight daily.

The following examples are provided in illustration of the invention and should not be construed in any way as constituting a limitation thereof.

### Example 1

#### Preparation of Oral Formulations of the Hydrochloride Salt of Compound A

Oral formulations of the hydrochloride salt of Compound A may be prepared substantially as described in GB-A-2127406.

### Example 2

#### Preparation of Parenteral Injections/Infusions of the Hydrochloride Salt of Compound A

| (i) Hydrochloride salt of Compound A equivalent to 50 mg base | 54mg | 54mg | 54mg |
|---|---|---|---|
| $\beta$-cyclodextrin hydrate | 143mg | 166mg | 238mg |
| Sodium hydroxide solution | to pH7 | to pH7 | to pH7 |
| Water suitable for injection | to 50ml | to 50ml | to 50ml |

The hydrochloride salt of Compound A was dissolved in 35ml water suitable for injection and the $\beta$-cyclodextrin was added. This solution was titrated to pH7 with 0.02M sodium hydroxide solution and then adjusted to volume with water suitable for injection.

The solution may then be sterilised by filtration and filled into vials or ampoules.

| (ii) Hydrochloride salt of Compound A equivalent to 50mg base | 54mg |
|---|---|
| $\beta$-cyclodextrin hydrate | 166mg |
| Sodium chloride | 450mg |
| pH7.0 phosphate buffer | 2.5ml |
| Sodium hydroxide solution | to pH7 |
| Water suitable for injection | to 50ml |

The hydrochloride salt of Compound A was dissolved in approximately 25ml water suitable for injection. The $\beta$-cyclodextrin was dissolved therein and the resulting solution was titrated to pH6 with 0.02M sodium hydroxide solution and the phospate buffer added. The sodium chloride was added to the solution and the pH adjusted to pH7 with sodium hydroxide. The solution was made up to volume with water suitable for injection. A sample of this solution was filled into a glass vial which was sealed with a rubber plug and metal overseal. This was then autoclaved.

## Claims

1. A combination comprising cyclosporin A and [1R]-[[α(Z),2β,3β, 5α]]-(+)-7-[5-[[1,1'-biphenyl)-4-yl]methoxy]-3-hydroxy-2-(1-piperidinyl)cyclopentyl]-4-heptenoic acid or a physiologically acceptable salt, solvate or cyclodextrin complex thereof.

2. A combination as claimed in Claim 1 for use as an active therapeutic agent.

3. A combination as claimed in Claim 1 for use in the preparation of a medicament for the management of graft rejection and auto immune diseases.

4. A combination as claimed in any one of Claims 1 to 3 wherein the heptenoic acid is in the form of the hydrochloride salt.

5. A combination as claimed in any one of Claims 1 to 3 wherein the heptenoic acid is in the form of a cyclodextrin complex.

6. [1R-[1α(Z),2β,3β,5α]]-(+)-7-[5-[[1,1'-biphenyl)-4-yl)methoxy)-3-hydroxy-2-(1-piperidinyl)cyclopentyl]-4-heptenoic acid for use as an immunosuppressive agent in association with cyclosporin A.

7. A pharmaceutical formulation comprising a combination as claimed in Claim 1 together with a pharmaceutically acceptable carrier therefor.

8. A pharmaceutical formulation as claimed in Claim 7 wherein the heptenoic acid is in the form of a hydrochloride salt.

9. A pharmaceutical formulation as claimed in Claim 7 wherein the heptenoic acid is in the form of a cyclodextrin complex.

10. [1R-[1α(Z),2β,3β,5α]]-(+)-7-[5-[[1,1'-biphenyl)-4-yl)methoxy)-3-hydroxy-2-(1-piperidinyl)cyclopentyl]-4-heptenoic acid for use in the manufacture of a medicament for the treatment of cyclosporin A-induced nephrotoxicity.